(19)

Europäisches
Patentamt

European
Patent Office

Office européen
des brevets

(11) **EP 3 597 595 A1**

(12) **EUROPEAN PATENT APPLICATION**

(43) Date of publication:
**22.01.2020   Bulletin 2020/04**

(51) Int Cl.:
***C01B 32/18*** (2017.01)          ***C01B 32/182*** (2017.01)
***C01B 32/198*** (2017.01)          ***A61K 8/04*** (2006.01)

(21) Application number: **18382533.0**

(22) Date of filing: **17.07.2018**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicant: **Graphenano Medical Care, S.L.**
**28006 Madrid (ES)**

(72) Inventors:
• **MARTÍNEZ ROVIRA, Martín**
  **E-30510 Yecla - Murcia (ES)**
• **MARTÍNEZ ROVIRA, José Antonio**
  **E-30510 Yecla - Murcia (ES)**
• **LAVÍN LÓPEZ, María del Prado**
  **E-30510 Yecla - Murcia (ES)**

• **ROMERO IZQUIERDO, Amaya**
  **E-13071 Ciudad Real (ES)**
• **RODRÍGUEZ PUYOL, Manuel**
  **E-28042 Madrid (ES)**
• **RODRÍGUEZ PUYOL, Diego**
  **E-28042 Madrid (ES)**
• **DE FRUTOS GARCÍA, Sergio**
  **E-28006 Madrid (ES)**
• **GRIERA MERINO, Mercedes**
  **E-28006 Madrid (ES)**
• **HATEM VAQUERO, Marco Antonio**
  **E-28006 Madrid (ES)**
• **OROZCO AGUDO, Ana Isabel**
  **E-28006 Madrid (ES)**

(74) Representative: **ABG Intellectual Property Law,
S.L.**
**Avenida de Burgos, 16D**
**Edificio Euromor**
**28036 Madrid (ES)**

(54)    **GRAPHENE PRODUCT AND COSMETIC USES THEREOF**

(57)    Graphene product obtained from Graphene Nanofibers (GNFs), Graphene Oxide or reduced Graphene Oxide (RGO), having a modified crystal structure and a defined size distribution. The product is non toxic and useful biological properties such as modification of the adipocytes phenotype. The product can be used in cosmetics.

**EP 3 597 595 A1**

**Description**

**FIELD OF THE INVENTION**

The invention relates to new graphene products, their compositions and their use in cosmetics.

**BACKGROUND OF THE INVENTION**

The family of graphene-based or graphene-related materials recently stepped into the spotlight after the 2010 Nobel Prize in Physics, and subsequent explosion in development of numerous applications for these materials in energy, electronics, sensors, light processing, medicine, and environmental fields. Graphene, the "founding" member of this family, is a two-dimensional material made of $sp^2$-hybridized carbon atoms arranged in a hexagonal honeycomb lattice.

The extended family of graphene-related materials includes graphene (single- and multilayered), graphite, polycyclic aromatic hydrocarbons, carbon nanotubes, fullerenes, various graphene nanostructures of different dimensionalities (e.g., graphene nanofibers, graphene nanoparticles, graphene quantum dots, graphene nanoribbons, graphene nanomeshes, graphene nanodisks, graphene foams, graphene nanopillars), any combinations of other graphene-related materials, substituted graphene-related materials (e.g., the substitution of carbon atoms with N, B, P, S, Si, or others), and graphene-related materials functionalized with reactive functional groups (e.g., carboxyl groups, esters, amides, thiols, hydroxyl groups, diol groups, ketone groups, sulfonate groups, carbonyl groups, aryl groups, epoxy groups, phenol groups, phosphonic acids, amine groups, porphyrin, pyridine, polymers and combinations thereof). Specific examples of graphene-related materials include graphene oxide (GO) and reduced graphene oxide (RGO).

Several publications describe the use of graphene materials for medical applications.

US2006/0134096 describes compositions and methods for medical use of graphene containing compositions, in particular non-porous carbon, other than fullerene or nanotubes, comprising graphene. They are used topically on wounds, as sorbent for toxins or in hemodialysis.

EP313353 discloses graphene nanostructure-based pharmaceutical compositions for preventing or treating neurodegenerative diseases. The graphene nanostructure inhibits fibril formation caused by protein misfolding.

US2014/0120081 discloses the use of carbon nanomaterials to treat oxidative stress in a subject by reducing the level of reactive oxygen species. The carbon nanomaterial is selected from nanotubes, graphene, graphene nanoribons, graphite, graphite oxide, etc. that can be functionalized.

GB2532449 describes functionalised nanomaterial for use in the treatment, prophylaxis, or prevention of cancer by inhibiting proliferation of cancer stem cells, wherein the nanomaterial is mono-layer graphene, few-layer graphene, nano-graphite, single-wall or multi-wall carbon nanotubes, fullerenes, carbon nanohorns, carbon nanofibres, or amorphous or partially amorphised nanocarbons or mixtures thereof. Graphene oxide is preferred.

Guranathan S. and Kim J-H. in International Journal of Nanomedicine, 2016:11, pages 1927-1945 review the synthesis, toxicity, biocompatibility and biomedical applications of graphene and graphene-related materials. As discussed in this document, many of these products still present problems of associated toxicity and biocompatibility. The toxic effect of graphene can be influenced by physicochemical properties such as size and distribution, surface charge, surface area, layer number, lateral dimensions, surface chemistry, purity, particulate state, surface functional groups and shape. Anticancer therapy, Photo-thermal therapy, drug delivery, gene transfection, biosensisng, imaging and tissue engineering are among the biomedical applications mentioned in this review.

There is still a need for new graphene based materials with low or no toxicity, good biocompatibility and able to provide a useful biological effect and applications in cosmetics.

**BRIEF DESCRIPTION OF THE INVENTION**

We have now found new graphene products having remarkable properties, low or no toxicity and useful in cosmetics.

In a first aspect the invention, is directed to a graphene nanomaterial selected from graphene nanofibers, graphene oxide, reduced graphene oxide, or combinations thereof, wherein the graphene nanomaterial has a particle size distribution having a dn(90) of 0.60 $\mu$m or smaller in number of particle and a dv(90) of 80.00 $\mu$m or smaller in volume particle, as measured by laser diffraction particle analyzer.

The graphene nanomaterial as defined above is preferably in the form of graphene nanofibers.

Preferably, the BET specific surface area of the graphene nanomaterial is comprised between 100 and 500 $m^2$/g, more preferably between 300-350 $m^2$/g.

In another embodiment, the pore volume of the graphene nanomaterial is between 0.35-0.40 $cm^3$/g.

It is also preferred that the impurities in the graphene nanomaterial are less than 0.01% by weight.

In another aspect, the invention is directed to the use of a graphene nanomaterial as defined as a cosmetic.

Preferably the cosmetic is for the treatment of the skin.

In another aspect, the invention is directed to the use of the above define graphene nanomaterial to to improve the appearance of the skin, preferably to reduce adipose tissue of the skin, preferably to reduce fat in the hypodermis.

In a further aspect, the invention is directed to a cosmetic treatment method for preventing or reducing the increase in the volume of hypodermal adipose tissue and/or the formation of fatty lumps, characterized in that the graphene nanomaterial according to any of claims 1-7, or a composition comprising it, is applied to the skin. Preferably, the cosmetic treatment is for the reduction of cellulitis, wrinkles and/or varicose veins.

Preferably, the cosmetic is for topical treatment of the skin.

In another aspect, the invention is directed to cosmetic compositions comprising the above define graphene nanomaterial.

**FIGURES**

**Figure 1.-** Raman spectroscopy of GMC-1

**Figure 2.-** Adsoprtion - desorption in $N_2$ of GMC-1

**Figure 3A.-** X-Ray diffraction of GNFs raw material

**Figure 3B.-** X-Ray diffraction of GMC-1

**Figure 4A.-** Comparison of the particle size distribution in number of particles for GNFs raw material and GMC-1.

**Figure 4B.-** Comparison of the particle size distribution according to the percentage of particle in volume of particles for GNFs raw material and GMC-1.

**Figure 5.-** Toxicity assays in cultured adipocytes (differentiated from 3t3l1 cell line) treated for 24 hours with 2x GMC-1 or vehicle (CT). A) Viability determined by trypan blue cellular exclusion and B) cellular metabolism determined by MTT with vehicle and 10x concentration of GMC-1. The values are represented as mean +/- SEM.

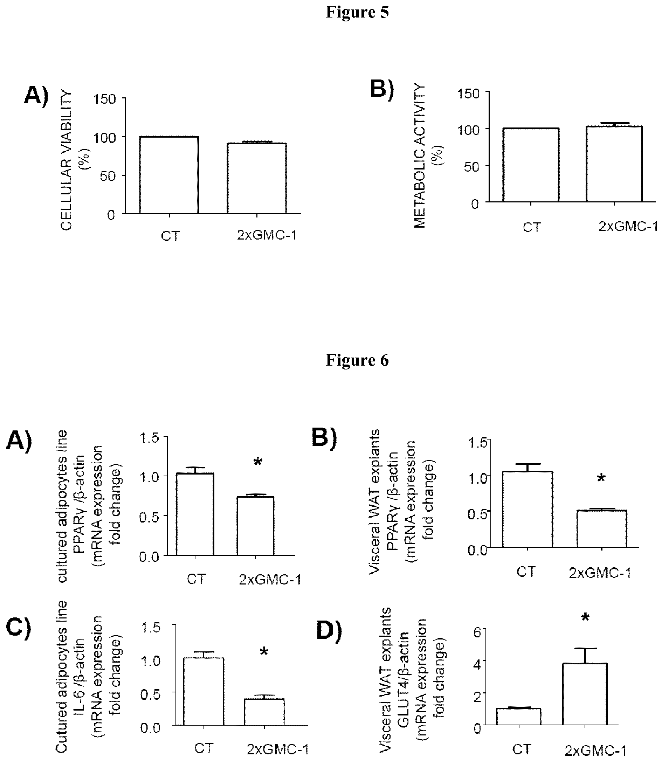

**Figure 6.-** Adipose differentiation markers expression in cultured fat models. Cultured adipocytes (differentiated from 3T3L1 cell line) or isolated visceral white adipose tissue explants from rodents (*Rattus norvegicus*) were treated for 24 hours with 2x GMC-1 or vehicle (CT). The mRNA expression fold change was determined by RT-qPCR for differentiation marker PPAPγ (A, B) and C) the inflammation adipokine IL-6 (C) and the insuline sensitivity marker GLUT4 (D) in visceral white adipose tissue. The values are represented as mean +/- SEM. *= P<0.05 vs. control, value considered statistically significant.

**DETAILED DESCRIPTION OF THE INVENTION**

Graphene raw materials

In the present invention, the term "graphene" refers to a material forming a polycyclic aromatic molecule with multiple carbon atoms covalently bonded to each other. The covalently bonded carbon atoms form a six-member ring as a repeating unit.

The term "graphene oxide" may be abbreviated as "GO" and may include a structure in which a functional group containing oxygen such as a carboxyl group, a hydroxyl group, or an epoxy group is bonded onto graphene.

The term "reduced graphene oxide" refers to graphene oxide decreased in a percentage of oxygen through a reduction process and can be abbreviated as "RGO".

The term "Graphene nanofibers" (GNFs) refers to cylindric nanostructures with graphene layers arranged as stacked cones, cups or plates. The graphene plane surface is canted from the fiber axis, which exposes the plane edges present on the interior and exterior surfaces of the carbon nanofibers.

The term "graphene nanotubes" (GNTs) refers to single wall or multi-wall concentric cylinders of graphene where the basal planes form a less reactive surface compared to that of Graphene Nanofibers because they are cylindrical and hollow like a tube but graphene nanofibers are like rods and generally there is no inner empty space within them.

The product of the invention is a carbon-based nanomaterial derived from GNF, GO or RGO that is subjected to a series of purifications and treatments to obtain a medical grade material having unexpected biological properties.

The starting carbon nanomaterial is a graphene-based material (graphene nanofibers, graphene oxide or reduced graphene oxide). Graphene nanofibers are especially preferred as starting material to prepare the product of the invention. In one embodiment, the graphene nanofibers used to prepare the product of the invention have a particle size distribution dn(90) of 4.0 $\mu$m or smaller for number of particle and a dv(90) of 105.00 $\mu$m or smaller for volume particle. Preferably they have a surface area of about 250-400 m$^2$/g.

Raw materials used to obtain the product of the invention can be synthesized following a wide variety of methods, such as epitaxial growth on Silicon Carbide, Chemical Vapor Deposition, micromechanical or mechanical exfoliation of graphite, chemical oxidation of graphite, reduction of graphite oxide using thermal, chemical or multistep reduction, catalysis decomposition of hydrocarbons over metal catalyst, unrolling carbon nanotubes, electrospinning, etc.

Epitaxial growth on Silicon Carbide is a method in which monolayers isolated from graphene can be synthesized on a monocrystalline silicon carbide crystal (SiC), which is used as a substrate. This method consists of heating SiC wafers to high temperatures (> 1100°C) and high vacuum. Under the conditions mentioned, the silicon atoms sublime obtaining the epitaxial growth of graphene on its surface (the carbon atoms rearrange themselves forming graphene) [Sutter, P., Epitaxial graphene: How silicon leaves the scene. Nature Materials, 2009. 8(3): p. 171-172.]

In the Chemical Vapor Decomposition method, a carbon source decays catalytically on a catalytic substrate. The catalytic surface causes, after the thermal decomposition of the hydrocarbons, the dissolution of the carbon atoms generated inside the metal [Jacobberger, R.M., et al., Simple Graphene Synthesis via Chemical Vapor Deposition. Journal of Chemical Education, 2015. 92(11): p. 1903-1907, Lavin-Lopez, M.P., et al., Thickness control of graphene deposited over polycrystalline nickel. New Journal of Chemistry, 2015. 39(6): p. 4414-4423].

The micromechanical exfoliation of graphite consists in the separation of the outermost layer of said solid in flakes by means of fine scraping, using a solid surface object or adhesive tape [Geim, A.K. and K.S. Novoselov, The rise of graphene. Nature Materials, 2007. 6(3): p. 183-191]. The mechanical exfoliation allows separating the sheets that form the suspended graphite in organic or aqueous solvents by means of ultrasound waves. The material obtained is of high quality, however, it is not of great industrial interest given its low yield and its high production cost [Lotya, M., et al., Liquid phase production of graphene by exfoliation of graphite in surfactant/water solutions. Journal of the American Chemical Society, 2009. 131(10): p. 3611-3620].

The Staudenmaier, Brodie or Hummers method and its modifications can perform chemical exfoliation [Chua, C.K. and M. Pumera, Chemical reduction of graphene oxide: A synthetic chemistry viewpoint. Chemical Society Reviews, 2014. 43(1): p. 291-312, Chua, C.K., et al., Graphite oxides: Effects of permanganate and chlorate oxidants on the oxygen composition. Chemistry - A European Journal, 2012. 18(42): p. 13453-13459, Marcano, D.C., et al., Improved synthesis of graphene oxide. ACS Nano, 2010. 4(8): p. 4806-4814, Lavin-Lopez, M.D.P., et al., Influence of different improved hummers method modifications on the characteristics of graphite oxide in order to make a more easily scalable method. Industrial and Engineering Chemistry Research, 2016. 55(50): p. 12836-12847]. It involves the chemical oxidation of graphite, using strong oxidants in an acid medium. In these methods, the oxidizing agents separate the graphite sheets by the covalent incorporation of oxygenated functional groups, such as hydroxyl or carboxyl, obtaining graphite oxide. Subsequently, the graphite oxide is exfoliated to graphene oxide to achieve the separation of its sheets. The graphite oxide can be exfoliated to graphene oxide and later reduced to reduced graphene oxide by different methods, such as thermal reduction, chemical reduction or multistep reduction [Lavin-Lopez, M.P., et al., Influence of the reduction strategy in the synthesis of reduced graphene oxide. Advanced Powder Technology, 2017. 28(12): p. 3195-3203]. Inside thermal reduction, thermal annealing consists on a rapid heating to exfoliate the graphite oxide to obtain reduced graphene oxide [McAllister, M.J., et al., Single sheet functionalized graphene by oxidation and thermal expansion of graphite. Chemistry of Materials, 2007. 19(18): p. 4396-4404]. As an alternative to thermal annealing, other unconventional heating sources have been tried to reduce or eliminate the functional groups of graphite oxide, emphasizing among them, microwave radiation [Zhu, Y., et al., Microwave assisted exfoliation and reduction of graphite oxide for ultracapacitors. Carbon, 2010. 48(7): p. 2118-2122] and photoreduction [Zhang, Y., et al., Direct imprinting of microcircuits on graphene oxides film by femtosecond laser reduction. Nano Today, 2010. 5(1): p. 15-20]. The reduction or removal of the oxygenated functional groups using chemical reducing agents is based on the chemical reaction of these with the graphene oxide. BnOH, hydrochloric acid, hydrazine or ascorbic acid can be used as chemical reduction agents. Solvothermal reduction is an emerging technique within the chemical reduction of graphene oxide. In a solvothermal process a series of chemical reactions are carried out in a given solvent under supercritical conditions. The physico-chemical properties of a solvent subjected to these conditions can improve the chemical diffusion of the species [Demazeau, G., Solvothermal processes: A route to the stabilization of new material. Journal of Materials Chemistry, 1999. 9(1): p. 15-18]. The reduction strategies explained above are carried out in a single stage. To improve these strategies, different research groups have proposed the reduction of oxygenated functional groups in multiple stages [Gao, W., et al., New insights into the structure and reduction of graphite oxide. Nature Chemistry, 2009. 1(5): p. 403-408].

A wide variety of methods can also be used to synthesize graphene nanofibers (GNFs), which are especially preferred to prepare the product of the invention. For example, the Chemical Vapor Deposition method for carbon nanofiber is a catalytic method in which a carbonaceous source is decomposed in the presence of a catalyst to grown GNFs. Transition metal catalytic particles such as iron, nickel, cobalt, and copper are used as catalyst. CVD process takes place at temperatures ranging between 500 to 1200°C [Martin-Gullon, I., et al., Differences between carbon nanofibers produced using Fe and Ni catalysts in a floating catalyst reactor. Carbon, 2006. 44(8): p. 1572-1580]. Electrospinning is an alternative production method of GNFs. In this method, the sol-gel process is used needing-a needle with a fine tip. For this, high voltage is applied to the drop of the needle, causing the solution to come out of the needle towards a target. When the surface tension is high enough for the solution, avoid entering a fine drop, a fibrous structure can be extracted and collected in the objective [Zhang, L., et al., A review: Carbon nanofibers from electrospun polyacrylonitrile and their applications. Journal of Materials Science, 2014. 49(2): p. 463-480].

The average diameters and lengths of the porous graphitic material which are used to prepare the composite of the invention are measured by Transmission Electron Microscopy (TEM).

Purification and Treatment

A graphene nanomaterial synthesized following the methods reported above is used as raw material to synthesize the graphene-based medical grade material of the invention. The raw graphene nanomaterial is then subjected to a purification process, preferably using a strong acid ($H_2SO_4$, HCl, HF, $HNO_3$, HBr, etc.), to remove any metal or impurity introduced in the graphene nanomaterial structure during the synthesis process. Any process able to remove impurities without affecting the properties of the graphene material can be used. Among the acids, Hydrochloric or Fluorhydric acid are especially preferred, but the skilled person will select the acid and conditions depending on the amount and type of impurities present. The purification process preferably takes place at low temperatures (20-50°C) for several hours (12-24 hours). If a solution is used for the purification process, the purified graphene nanomaterial can then be washed with Millipore water until neutral pH and then dried, for example vacuum dried.

The purified graphene nanomaterial is also treated to achieve a reduced particle size distribution, which makes the product suitable for medical and cosmetic uses. The purified graphene nanomaterial is for example subjected to a process to reduce its size and modify its properties. In one embodiment it is subjected to an exfoliation process at room temperature, for example through ultrasonication, wet milling or hybrid processes. After that a delimitation process to control the particle sized between 10-100 $\mu$m takes place.

Finally, to control that the grade material is not dragging traces of other toxic compounds, including bacteriological contamination or endotoxins and in order to maintain asepsis and sterility conditions, the material can also be subjected to a standard depyrogenization process by heat, preferably at 200-500 ° C for 10-60 min.

Therefore, a further object of the invention is a method to prepare the product of the invention from raw graphene nanofibers, graphene oxide or reduced graphene oxide, comprising the following steps:

a) Purifying the raw graphene material, preferably using a strong acid, to remove any metal or impurity present in the graphene raw material,

b) Reducing the particle size of the purified graphene nanomaterial, preferably through an exfoliation process, to a particle size distribution having a dn(90) of 0.60 $\mu$m or smaller in number of particle and a dv(90) of 80.00 $\mu$m or smaller in volume particle, as measured by laser diffraction particle analyzer,

c) Optionally subjecting the obtained product to a depyrogenization process.

Product

The product of the invention is a purified graphene nanomaterial with a particle size distribution having a dn(90) of around 0.60 $\mu$m of smaller in number of particle and a dv(90) of 80.00 $\mu$m or smaller, preferably 70.00 $\mu$m or smaller in volume particle.

The particle size distribution is measured by a laser diffraction particle size analyzer. A particle Size Distribution D50 is the value of the particle diameter at 50% in the cumulative distribution. If D50 have a certain value, then 50% of the particles in the sample are larger than this value and 50% are smaller. The particle size distribution is the number of particles that fall into each of the various size ranges given as a percentage of the total number of all sizes in the sample of interest. The most widely used method of describing particle size distributions are d values (d10, d50 and d90) which are the intercepts for 10%, 50% and 90% of the cumulative mass.

These values can be thought of as the diameter of the material which divides the samples mass into a specified percentage when the particles are arranged on an ascending mass basis. The d10 is the diameter at which 10% of the sample's mass is comprised of particles with a diameter less than this value. The d50 is the diameter of the particle that 50% of a sample's mass is smaller than and 50% of a sample's mass is larger than. d90 is the diameter at which 90% of the sample's mass is comprised of particles with a diameter less than this value. These values can be applied for number of particles (dn) and volume of particles (dv).

A distribution having a dn(90) in number of particle means the point in the size distribution, up to and including which, 90% of the total number of material in the sample is contained.

A distribution having a dv(90) in volume of particle means the point in the size distribution, up to and including which, 90% of the total volume of material in the sample is contained.

The particle size distribution of the product of the invention is measured with a Mastersizer 3000 of Malvern Panalytical.

In the context of the present invention, the term "specific surface area (SSA)" refers to the total surface area of a material per unit of mass.

The properties of porosity and specific surface area described in the present application are measured using Brunnauer-Emmet-Teller ("BET") methods, applied in physical adsorption technique using nitrogen as the adsorptive material, which is well known to the person skilled in the art.

In an embodiment of the invention, the BET surface area of the product of the invention is between 300-350 $m^2/g$.

In another embodiment, the pore volume of the product of the invention is between 0.35-0.40 $cm^3/g$.

In a preferred embodiment, the product of the invention has a BET surface area between 300-350 $m^2/g$ and a pore volume between 0.35-0.40 $cm^3/g$.

In a preferred embodiment, the product of the invention is in the form of graphene nanofibers.

Composition

In another aspect, the invention relates to a cosmetic composition comprising the graphene product of the invention and one or more cosmetically acceptable excipients.

As used herein, the term "cosmetic composition" means a composition which is intended to be applied onto the consumer's skin, so as to regulate the condition of the skin and/or to improve the appearance of the skin, including a reduction of the fat in the hypodermis.

The cosmetic composition of the invention may include, in addition to the graphene product according to the present invention, which is an active ingredient, a conventional auxiliary excipient, such as a stabilizer, a solubilizing agent, a vitamin, a pigment, and a perfume.

The cosmetic composition may be prepared in any formulation that is conventionally used in the art. For example, the cosmetic composition may be prepared in the formulation of, for example, suspension, emulsion, paste, gel, cream, lotion, powder, oil, powder foundation, emulsion foundation, wax foundation, and spray, but the formulation thereof is not limited thereto. That is, the cosmetic composition may be prepared in the formulation of sun cream, softening cosmetic water, convergence cosmetic water, nutrition cosmetic water, nutrition cream, massage cream, essence, eye cream, pack, spray, or powder.

The term "excipient" refers to a vehicle, diluent or adjuvant that is administered with the active ingredient. The cosmetic excipients can be sterile liquids, such as water and oils, including those of petroleum, animal, vegetable or synthetic origin, such as peanut oil, soybean oil, mineral oil, sesame oil and similars.

In a preferred embodiment the cosmetic composition of the invention is suitable for topical administration to the skin, such as for example creams, lotions, ointments, microemulsions, fatty ointments, gels, emulsion-gels, pastes, foams, tinctures, solutions, patches, bandages and transdermal therapeutic systems. Most preferred are creams, hydrogels or emulsion-gels.

Creams or lotions are oil-in-water emulsions. Oily bases that can be used are fatty alcohols, especially those containing from 12 to 18 carbon atoms, for example lauryl, cetyl or stearyl alcohol, fatty acids, especially those containing from 10 to 18 carbon atoms, for example palmitic or stearic acid, fatty acid esters, e.g. glyceryl tricaprilocaprate (neutral oil) or cetyl palmitate, liquid to solid waxes, for example isopropyl myristate, wool wax or beeswax, and/or hydrocarbons, especially liquid, semi-solid or solid substances or mixtures thereof, for example petroleum jelly (petrolatum, Vaseline) or paraffin oil. Suitable emulsifiers are surface-active substances having predominantly hydrophilic properties, such as corresponding non-ionic emulsifiers, for example fatty acid esters of polyalcohols and/or ethylene oxide adducts thereof, especially corresponding fatty acid esters with (poly)ethylene glycol, (poly)propylene glycol or sorbitol, the fatty acid moiety containing especially from 10 to 18 carbon atoms, especially partial glycerol fatty acid esters or partial fatty acid esters of polyhydroxyethylene sorbitan, such as polyglycerol fatty acid esters or polyoxyethylene sorbitan fatty acid esters (T weens), and also polyoxyethylene fatty alcohol ethers or fatty acid esters, the fatty alcohol moiety containing especially from 12 to 18 carbon atoms and the fatty acid moiety especially from 10 to 18 carbon atoms, such as polyhydroxyethyleneglycerol fatty acid ester (for example Tagat S), or corresponding ionic emulsifiers, such as alkali metal salts of fatty alcohol sulfates, especially having from 12 to 18 carbon atoms in the fatty alcohol moiety, for example sodium lauryl sulfate, sodium cetyl sulfate or sodium stearyl sulfate, which are usually used in the presence of fatty alcohols, for example cetyl alcohol or stearyl alcohol. Additives to the aqueous phase are, inter alia agents that prevent the creams from drying out, for example humectants, such as polyalcohols, such as glycerol, sorbitol, propylene glycol and/or polyethylene glycols, and also preservatives, perfumes, gelling agents, etc.

Ointments are water-in-oil emulsions that contain up to 70%, but preferably from approximately 20% to approximately 50%, water or aqueous phase. Suitable as fatty phase are especially hydrocarbons, for example petroleum jelly, paraffin oil and/or hard paraffins, which, in order to improve the water-binding capacity, preferably contain suitable hydroxy compounds, such as fatty alcohols or esters thereof, for example cetyl alcohol or wool wax alcohols, or wool wax or beeswax. Emulsifiers are corresponding lipophilic substances, for example of the type indicated above, such as sorbitan fatty acid esters (Spans), for example sorbitan oleate and/or sorbitan isostearate. Additives to the aqueous phase are, inter alia humectants, such as polyalcohols, for example glycerol, propylene glycol, sorbitol and/or polyethylene glycol, and also preservatives, perfumes, etc.

Microemulsions are isotropic systems based on the following four components: water, a surfactant, for example a tensioactive, a lipid, such as a non-polar or polar oil, for example paraffin oil, natural oils such as olive or maize oil, and an alcohol or polyalcohol containing lipophilic groups, for example 2-octyldodecanol or ethoxalated glycerol or polyglycerol esters. If desired, other additives may be added to the microemulsions. Microemulsions have micelles or particlaes with sizes below 200 nm and are transparent or translucid systems, the form spontaneoulsy and are stable. Fatty ointments are water- free and contain as base especially hydrocarbons, for example paraffin, petroleum jelly and/or liquid paraffins, also natural or partially synthetic fat, such as fatty acid esters of glycerol, for example coconut fatty acid triglyceride, or preferably hardened oils, for example hydrogenated groundnut oil, castor oil or waxes, also fatty acid partial esters of glycerol, for example glycerol mono- and di-stearate, and also, for example, the fatty alcohols increasing the water-absorption capacity, emulsifiers and/or additives mentioned in connection with the ointments.

In relation to gels, a distinction is made between aqueous gels, water-free gels and gels having a low water content, which gels consist of swellable, gel-forming materials. There are used especially transparent hydrogels based on inorganic or organic macromolecules. High molecular weight inorganic components having gel-forming properties are predominantly water-containing silicates, such as aluminium silicates, for example bentonite, magnesium aluminium silicates, for example Veegum, or colloidal silicic acid, for example Aerosil. As high molecular weight organic substances there are used, for example, natural, semisynthetic or synthetic macromolecules. Natural and semi- synthetic polymers are derived, for example, from polysaccharides containing a great variety of carbohydrate components, such as celluloses, starches, tragacanth, gum arabic and agar-agar, and gelatin, alginic acid and salts thereof, for example sodium alginate, and derivatives thereof, such as lower alkylcelluloses, for example methyl- or ethyl-cellulose, carboxy- or hydroxy-lower alkylcelluloses, for example carboxymethyl- or hydroxyethyl-cellulose. The components of synthetic gel-forming macromolecules are, for example, suitably substituted unsaturated aliphatic compounds such as vinyl alcohol, vinylpyrrolidine, acrylic or methacrylic acid.

Emulsion-gels - also called "emulgels" - represent topical compositions which combine the properties of a gel with those of an oil-in-water emulsion. In contrast to gels, they contain a lipid phase which due to its fat-restoring properties enables the formulation to be massaged in whilst, at the same time, the direct absorption into the skin is experienced as a pleasant property. Furthermore, one can observe an increased solubility for lipophilic active ingredients. One advantage of emulsion-gels over oil-in-water emulsions resides in the enhanced cooling effect which is brought about by the coldness due to evaporation of the additional alcohol component, if present.

Foams are administered, for example, from pressurised containers and are liquid oil-in water emulsions in aerosol form; unsubstituted hydrocarbons, such as alkanes, for example propane and/or butane, are used as propellant. As oil phase there are used, inter alia hydrocarbons, for example paraffin oil, fatty alcohols, for example cetyl alcohol, fatty acid esters, for example isopropyl myristate, and/or other waxes. As emulsifiers there are used, inter alia, mixtures of emulsifiers having predominantly hydrophilic properties, such as polyoxyethylene sorbitan fatty acid esters (Tweens), and emulsifiers having predominantly lipophilic properties, such as sorbitan fatty acid esters (Spans). The customary additives, such as preservatives, etc., are also added. Tinctures and solutions generally have an ethanolic base, to which water may be added and to which there are added, inter alia, polyalcohols, for example glycerol, glycols and/or polyethylene glycol, as humectants for reducing evaporation, and fat- restoring substances, such as fatty acid esters with low molecular weight polyethylene glycols, propylene glycol or glycerol, that is to say lipophilic substances that are soluble in the aqueous mixture, as a replacement for the fatty substances removed from the skin by the ethanol, and, if necessary, other adjuncts and additives. Suitable tinctures or solutions may also be applied in spray form by means of suitable devices.

Transdermal therapeutic systems with -in particular- local delivery of the graphene product of the invention contain an effective amount of the graphene product optionally together with a carrier. Useful carriers comprise absorbable pharmacological suitable solvents to assist passage of the active ingredient through the skin. Transdermal delivery systems are, for example, in the form of a patch comprising (a) a substrate (= backing layer or film), (b) a matrix containing the active ingredient, optionally carriers and optionally (but preferably) a special adhesive for attaching the system to the skin, and normally (c) a protection foil (= release liner). The matrix (b) is normally present as a mixture of all components or may consist of separate layers.

Membranes and matrixes comprising the graphene product of the invention are also suitable for the topical application of the product, either by themselves or as a part of a more complex product, such as a wound dressing, a bandage, etc. Example of such membranes or matrixes are natural polymers such as polysaccharides (alginates, chitin, chitosan, heparin, chondroitin, carragenaan), proteoglycans and proteins (collagen, gelatin, fibrin, keratin, silk fibroin, eggshell membrane); synthetic polymers such as hydrogels or biomimetic extracellular matrix micro/nanoscale fibers based on polyglycolic acid, polylactic acid, polyacrylic acid, poly-e-caprolactone, polyvinylpyrrolidone, polyvinyl alcohol, polyethylene glycol, etc.

All these systems are well known to the person skilled in the art. The manufacture of the topically administrable pharmaceutical or cosmetic preparations is effected in a manner known per se, for example by suspending the graphene product of the invention in the base or, if necessary, in a portion thereof.

The compositions according to the invention may also comprise conventional additives and adjuvants for dermatological applications, such as preservatives, especially paraben esters like methylparaben, ethylparaben, propylparaben, butylparaben, or quaternary ammonium compounds like benzalkonium chloride, or formaldehyde donors like imidazonidinyl urea, or alcohols like benzyl alcohol, phenoxyethanol or acids like benzoic acid, sorbic acid; acids or bases used as pH buffer excipients; antioxidants, especially phenolic antioxidants like hydroquinone, tocopherol and derivatives thereof, as well as flavonoids, or miscellaneous antioxidants like ascorbic acid,ascorbyl palmitate; perfumes; fillers such as kaolin or starch; pigments or colorants; UV-screening agents; moisturizers, especially glycerin, butylen glycol, hexylen glycol, urea, hyaluronic acid or derivatives thereof; anti-free radical agents such as vitamin E or derivatives thereof; penetration enhancers especially propylene glycol; ethanol; isopropanol; dimethylsulfoxide; N-methyl-2- pyrrolidone; fatty acids/alcohols such as oleic acid, oleyl alcohol; terpenes such as limonen, menthol, 1-8 cineole; alkyl esters such as ethyl acetate, butyl acetate; ion pairing agents such as salicylic acid.

This composition can constitute a mask, a cleansing, protecting, treating or care cream for the face or for the body (for example day creams, night creams, make-up removing creams, foundation creams or antisun creams), a make-up removing milk or a lotion, gel or foam for caring for the skin, such as a cleansing lotion.

Further details concerning suitable topical formulations may be obtained by reference to standard textbooks such as "Harry's Cosmeticology" 9th Edition (2015), Chemical Publishing Co.

The amount of the graphene nanomaterial product of the invention in the formulation can be in the range of 0.01% to 10% w/w, preferably from 0.01% to 5% w/w, more preferably from 0.1% to 3% w/w.

Beneficial Effects and uses

As evidenced by the examples below, the product of the invention is not toxic, also in the case of topical applications, and has good biocompatibility.

In addition, and surprisingly, the product of the invention substantially modifies adipose tissue tissue phenotype *in vitro* and *ex vivo,* and therefore is useful as fat reducer in cosmetic applications.

Therefore, the graphene product of the invention, and compositions containing it, are useful as cosmetics. The term "cosmetic" is understood to mean intended to improve the aesthetic appearance of the skin or its appendages. It is non-therapeutic.

Plumpness and/or excess weight are related to the changes in the phenotype, and so for the response, of certain cells, known as adipocytes, where the dynamic process to accumulate or liberate the free fatty acids and glycerol that form the triglycerides is disbalanced. [Saponaro C et al. Nutrients 2015, 7, 9453-9474; Tontonoz P. et al. Annu. Rev. Biochem. 2008. 77:289-312]

In the case of an excessively rich diet or for people not pursuing physical exercise, a substantial imbalance of the fat accumulated is established in the body, which includes the increase of the adipose tissue areas, and may be gradually reflected by deformation of the skin brought about by the thickening of the hypodermis in which the subcutatenous adipose tissue is found.

The graphene product of the invention has proved to be effective in the modification of the phenotype plasticity of cultured adipocytes and fat depots, both *in vitro* and *ex vivo.*

Therefore, the graphene product of the invention, and cosmetic compositions comprising it, are useful in the cosmetic treatment of fat deposits in the skin and reduction of cellulitis and wrinkles

In view of the above, the invention is also directed to a cosmetic treatment method intended to prevent or reduce the increase in the volume of adipose tissue and/or the formation of fatty lumps and/or a slimming method comprising the application, over all or part of the body, of a composition comprising the graphene product of the invention. Application can be carried out over regions subject to lipodystrophia, such as the abdomen, the top of the thighs or arms, or certain regions of the face, such as the bottom of the face. The method improves the appearance of the person and skin by modifying the adipocytes phenotype.

Therefore, the graphene product of the invention may be used in non-therapeutic (e.g. cosmetic) treatments ("non-therapeutic use"), e.g. to enhance the fat distribution for aesthetic effects, e.g. the fat distribution at limbs, the abdomen and/or the buttocks. Among the cosmetic uses of the graphene product of the invention are the reduction of subcutaneous fat, which would improve the appearance of skin with cellulitis or other deformations due to the accumulation of fat in the hypodermis, elimination of wrinkels, varicose veins and other skin defects or appearance susceptible to be improved.

Cellulite is localized in the dermis. In the hypodermis, a deep layer of the dermis, the adipose cells, or adipocytes, gather together to form lobules delimited by partitions (rows of collagen fibers) that are parallel to each other and perpendicular to the skin surface. The adipocyte is a large cell, 80% of the volume of which consists of one or more lipid vacuoles. It is of variable size. If the vacuole is overloaded with fat, the volume of the adipocyte increases and the dermal connective tissue thickens.

Another effect of fat accumulation in adipocytes is the reduction of blood reaching tissues which can result in liquid accumulation, swelling, tissue damage and necrosis. This has negative effects on the body and general appearance, such as for example the formation of varicose veins. Therefore, the product of the invention and compositions comprising it are useful in preventing or repairing this damage. Preferably, the product of the invention is useful in the cosmetic treatment of varicose veins.

In another embodiment, the graphene product of the invention, and cosmetic compositions comprising it, are useful in cosmetic skin repair.

Mode of administration

The composition used in the present method is preferably applied topically.

The daily dosage of the topical formulation comprising the graphene product of the invention may depend on various factors, such as sex, age, weight and individual condition of the patient.

**EXAMPLES**

**Example 1:**

**GMC-1 PREPARATION AND PHYSICO--CHEMICAL CHARACTERIZATION**

Raw Materials

The textural characteristics, the degree of graphitization, and the main physical-chemical and thermal properties of the raw materials that can be used to prepare the graphene nanomaterials of the invention are present in Table 1 below.

*Table 1: Physico-chemical properties of raw GNFs, GO and RGO*

| Characteristics | Property | GNFs | GO | RGO |
|---|---|---|---|---|
| Textural | Surface area ($m^2/g$) | 250-400 | 25-35 | $\approx 400$ |
| Graphitization degree | DRX: npg [a] (npg from graphite$\approx$95) | 6-10 | 6-9 | 4-6 |
| | RAMAN: $I_D/I_G$ ($I_D/I_G$ from graphite$\approx$0.6)[b] | 1.0-1.4 | 0.9-1.0 | 0.8-0.8 |
| Physical and Chemical | Odour, colour and appearance | Without smell, black powder, spongy | Without smell, black powder, spongy | Without smell, black powder, spongy |
| | Solid content | 100% | 100% | 100% |
| | Solubility | | Polar solvent | Alcohols, water, NMP, DMF |
| | Thermal conductivity (W/mK) | 1400-1600 | | |

(continued)

| Characteristics | Property | GNFs | GO | RGO |
|---|---|---|---|---|
| Thermal | Oxidation temperature (°C) | 680 | 520 | 480 |
| | Thermal decomposition/ oxidation products | Principally CO and $CO_2$ | Principally CO and $CO_2$ | Principally CO and $CO_2$ |
| [a]number of graphene planes in the crystal (npg = Lc/d); d is the interlaminate range; Lc is the average size of the crystal in the sample, in a perpendicular direction to the basal planes of graphene [b]$I_D/I_G$: quotient between the intensities of D and G bands in the RAMAN spectrum. | | | | |

In the present example graphene nanofibers (GNFs) have been used to prepare a material according to the invention.

The raw graphene-based carbon nanomaterial (GNFs) is subjected to a purification process using HF to remove metal and impurities introduced in the GNFs structure during the synthesis process. The purification process takes place at low temperatures (20-50°C) for several hours (12-24 hours). After that the purified carbon nanomaterial is vacuum dried and washed with Millipore water until neutral pH.

The purified GNFs were then subjected to an exfoliation process for several hours (2-5 h) at room temperature in a solution with water or other solvents. Finally, the material is subjected to a standard depirogenization process by heat (200-500 ° C for 10-60 min).

The resulting product (GMC-1) has been characterized as follows:

## Elemental Analysis of GMC-1

The main difference between GMC-1 and the raw materials can be observed in their elemental analysis (Table 2). In comparison with the raw GNFs, GMC-1 is only composed of carbon and oxygen. It does not have any impurity traces that could be harmful to human health, as confirmed by toxicology experiments.

*Table 2: Elemental analysis of raw CNFs, GrO, RGO and GMC-1*

| Element | CNFs | GrO | RGO | GMC-1 |
|---|---|---|---|---|
| C | 80-90 | 38-55 | 77-87 | 92-95 |
| O | 10-15 | 41-52 | 13-22 | 5-6 |
| Impurity traces (metals, catalyst support, etc.) | 0.5-1.5 | 1-10 | 1-5 | 0.0-0.01 |

## Raman spectroscopy of GMC-1

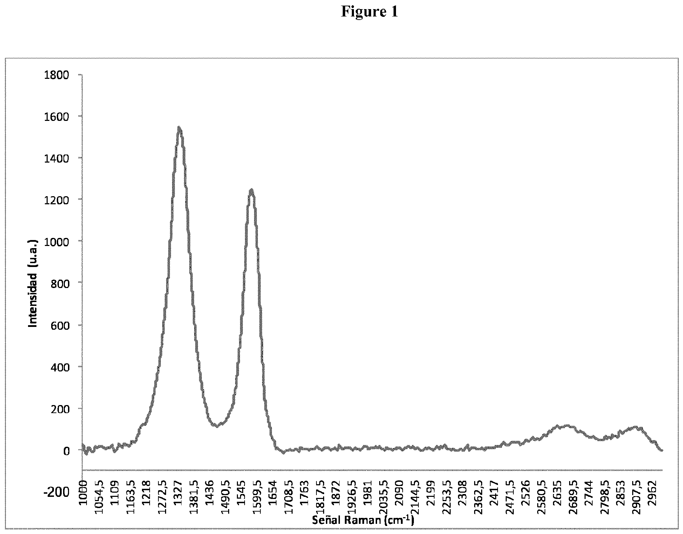

The Raman spectrum of GMC-1 was obtained using a 512 nm laser. It shows (**Figure** 1) the characteristic peaks of coal materials. Peak D, 1332 $cm^{-1}$, and peak G, 1580 $cm^{-1}$. The G band corresponds to the networks of carbon atoms, that is, to the ideal graphitic structure, while the D band is due to the existence of defects, both in the basal plane and at the edges. Graphene nanofibers have a band D of great intensity, greater than the band G. A large D peak can appear in graphitic materials if they contain a large number of edges, as in the case of these nanofibers. The fact that both peak D and G do not have too high a bandwidth also shows the crystallinity of the nanofibers.

## Absorption-Nitrogen Desorption Analysis of GMC-1

The basis for measuring the total surface area of a solid by physisorption of a gas is to detect the number of gas molecules needed to cover the surface of the solid. Once the area occupied by the molecule is known, the surface area of the solid can be estimated from the number of molecules of gas absorbed, measured volumetrically or gravimetrically (Brunauer, Emmett and Teller).

The total surface area was calculated by the multipoint BET equation, while the total pore volume was determined by the amount of vapor adsorbed at a relative pressure $P / P_0 = 0.99$, assuming that the pores are subsequently filled with liquid adsorbate. The average pore size, assumed to be cylindrical, was estimated from the total pore volume value and the surface area, assuming that pores that were not filled at relative pressures less than 1 did not contribute to the volume and surface area of the pore sample.

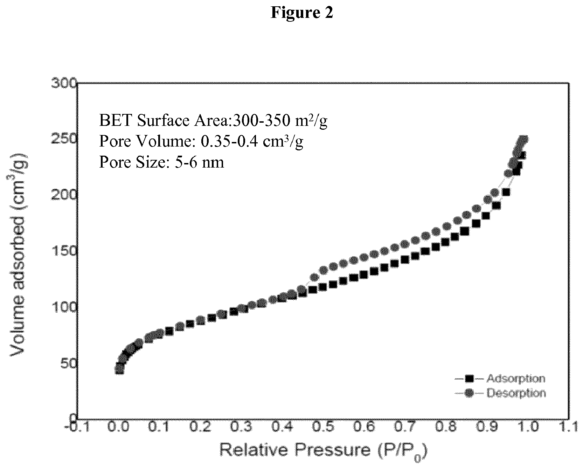

The analysis of surface area, pore volume and pore area were made by adsorption-desorption of $N_2$ at 77 K, using a QUANTACHROM model QUADRASORB SI model, with six degassing ports and three analysis ports, controlled with software (QUADRAWIN) that collects the values of relative pressure for each volume of $N_2$ dosed. **Figure 2** shows the surface area, pore volume and pore size of GMC-1.

BET Surface Area: 300-350 m²/g

Pore Volume: 0,35-0,4 cm³/g

Pore Size: 5-6 nm

## X-Ray Difraction of GMC-1

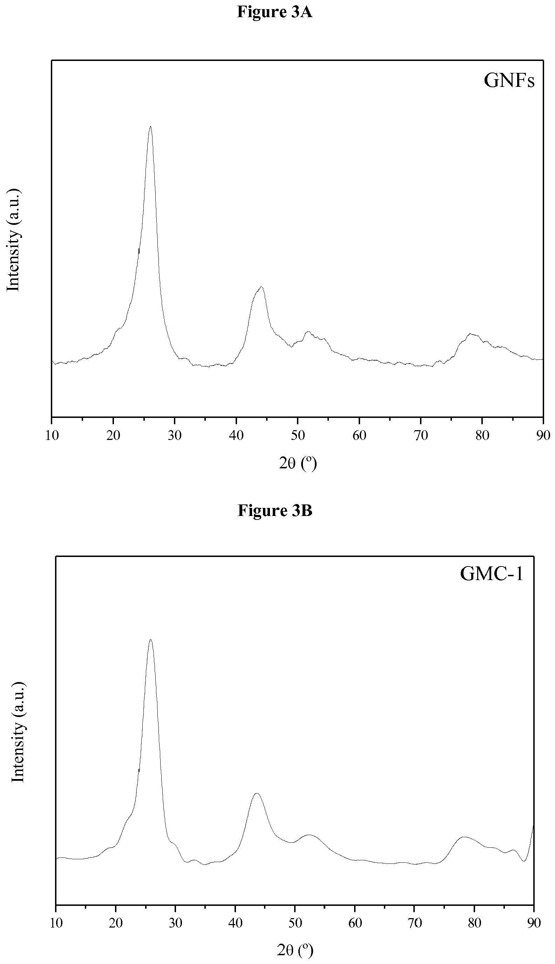

X-Ray diffractogram corresponding to a sample of GNFs was performed (**Figure 3A**). As it is observed, it presents a peak around 25.9° that corresponds to the distance between planes 002 of the graphite, or distance between the sheets of graphene. In the highly crystalline graphite, the interlaminar distance is 0.334 nm. In this case, the nanofibers have a slightly greater distance, 0.343 nm, which is indicative that they have a short-range crystallinity and are turbostratic. The crystal size in the direction perpendicular to the plane 002 (Lc) is 4.64 nm, indicative of the above mentioned fact.

**Figure 3B** shows the diffractogram corresponding to a sample of the material according to the invention GMC-1. GNFs and GMC-1 presented the same peaks, however in GMC-1 these peaks presented lower value of $2\theta$.

Table 3 shows the characteristic crystallographic parameters for GNFs and GMC-1:

Interlaminar space (d002)

Crystal stack height (Lc)

In-plane crystallite size (La)

Number of graphene layers in the crystal (npg)

$$d = \frac{\lambda}{2 \cdot sen\theta_1} \; ; \; L_c = \frac{k_1 \cdot \lambda}{FWHM \cdot cos\theta_1} \; ; \; La(nm) = \frac{k_2 \cdot \lambda}{FWHM \cdot cos\theta_2} \; ; \; npg = \frac{L_c}{d}$$

where:

$\lambda$, radiation wavelength ($\lambda$, =0,15404 nm)

$\theta_1$, diffraction peak position (°)

$\theta_2$, diffraction peak position (°)

k1, form factor (k=0,9)

k2, Warren Form Factor constant (k=1,84)

FWHM, width at half height of the corresponding diffraction peak (rad)

## Table 3: X-Ray Diffraction parameters of GNFs and GMC-1

|  | Lc (nm) | La (nm) | d (nm) | npg |
|---|---|---|---|---|
| GNFs | 2.19 | 2.99 | 0.341740 | 6.4 |
| GMC-1 | 2.08 | 2.8 | 0.344338 | 6.05 |

When GNFs are transformed into GMC-1 the crystal structure of the material changes. In this way, a decreased of the crystal stack height (Lc), the in-plane crystallite size (La) and the number of graphene layers in the crystal (Nc) is observed in GMC-1 due to the purification, cleaning and exfoliation process to which the material has been subjected. The interlaminar space in GMC-1 increases due to the exfoliation process experimented in the material. Crystal stack height (Lc) and in-plane crystallite size (La) experiment a decreased due to the purification and exfoliation process.

**Particle Size distribution**

The particle size distribution for the raw material GNFs and the product according to the invention GMC-1 was measured with a Mastersizer 3000 of Malvern Panalytical. The Mastersizer 3000 uses the technique of laser diffraction to measure the size of particles. It does this by measuring the intensity of light scattered as a laser beam passes through a dispersed particulate sample. This data is then analyzed to calculate the size of the particles that created the scattering pattern.

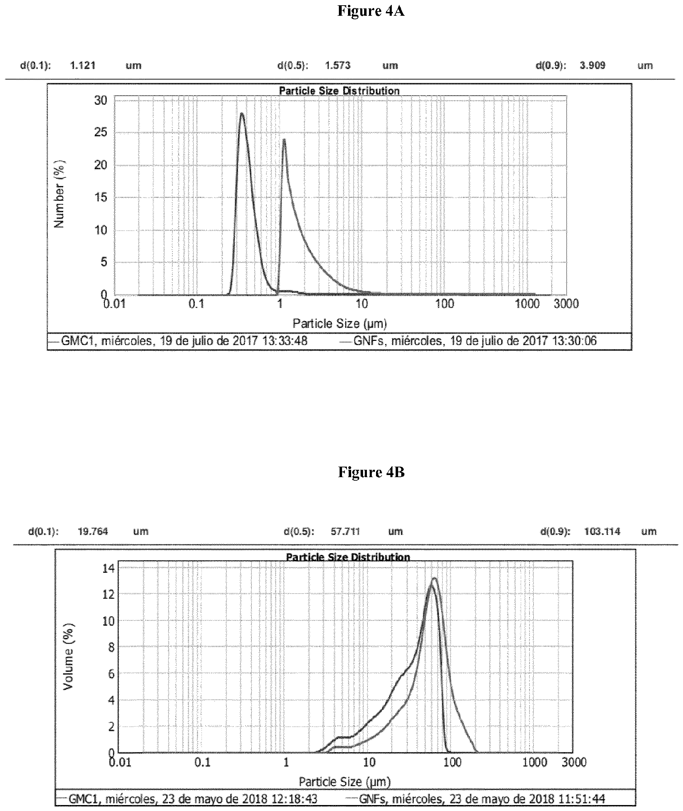

**Figure 4A** shows the comparison of the particle size distribution in number of particles for GNFs and GMC-1.

**Figure 4B** shows the comparison of the particle size distribution according to the percentage of particle in volume of particles for GNFs and GMC-1.

The parameters d(0.1), d(0.5) and d(0.9) can be observed in the Figures , dn is referred to the number of particles and dv is referred to the volume in the particles.

For GNFs, dn(10) parameter means that the 10% of the number of particles have a size of 1.121 $\mu$m or smaller, dn(50) parameter means that the 50% of the number of particle have a size of 1.573 $\mu$m or smaller and dn(90) parameter means that the 90% of the number of particle have a size of 3.909 $\mu$m or smaller. For GNFs, dv(10) parameter means that the 10% of the volume of the sample is occupied by particles with a size of 19.764 $\mu$m or smaller, dv(50) parameter means that the 50% of the volume of the sample is occupied by particles with a size of 57.711 $\mu$m or smaller and dv(90) parameter means that the 90% of the sample is occupied by particles with a size of 103.114 $\mu$m or smaller.

In the case of GMC-1, dn(10) parameter means that the 10% of the number of particles have a size of 0.313 $\mu$m or smaller, dn(50) parameter means that the 50% of the number of particle have a size of 0.394 $\mu$m or smaller and dn(90) parameter means that the 90% of the number of particle have a size of 0.577 $\mu$m or smaller. For GMC-1, dv(10) parameter means that the 10% of the volume of the sample is occupied by particles with a size of 10.549 $\mu$m or smaller, dv(50) parameter means that the 50% of the volume of the sample is occupied by particles with a size of 39.693 $\mu$m or smaller and dv(90) parameter means that the 90% of the sample is occupied by particles with a size of 69.576 $\mu$m or smaller.

The comparison in particle size distribution in number and volume between GNFs and GMC-1 shows that GMC-1 has a lower particle size distribution. Summarizing, dn(90) for GNFs has a size of 3,909 $\mu$m or smaller for number of particle and a dv(90) of 103,114 $\mu$m or smaller for volume particle whereas dn(90) of GMC-1 has a size of 0.577 $\mu$m or smaller in number of particle and a dv(90) of 69.576 $\mu$m or smaller in volume particle. Figures 4A and 4B show clearly these reduction on number and volume of particle in GMC-1 as compared with the raw material used (GNFs).

**BIOLOGICAL ACTIVITY**

Statistics

The data shown are represented as the means $\pm$ SEMs of a variable number of experiments. Student's t test was used for 2 samples and 1- or 2-way analysis of variance was used for >2 samples (ANOVA), with a paired or unpaired design followed by a multiple comparison test. Values of $P < 0.05$ were considered statistically significant.

**Example 2: TOXICITY ASSAY OF GMC-1 ON ADIPOCYTES**

Adipose tissue is distributed in different depots, from subcutaneous areas to the abdominal cavity (visceral fat). The tissue is formed by fat cells, or adipocytes, but also by pre-adipocytes and immune cells. The main function of the adipose tissue is to modulate and metabolize the triglycerides and to produce regulatory hormones. Besides the localization of the depots, traditionally the adipose tissue is divided in A) white adipose tissue which main function is to accumulate lipidic drops as energetic reserve, B) brown adipose tissue which main function is thermogenesis through their numerous mitochondria and C) the beige adipose tissue , which is present in WAT but with morphological and functional characteristics of BAT. Taking in consideration the differentiation state of the adipocytes from these different depots, from fully mature adipocytes to non-differentiated adipocytes, their expression patterns are considered to be modifiable and therefore these are cells with important phenotype plasticity [Luo L., Liu M.; Adipose tissue in control of metabolism. J Endocrinol. 2016 Dec;231(3):R77-R99.].

Before studying the functional activity of GMC-1, we first tested the product in suspension over the viability and toxicity in cultured adipocytes obtained by manipulation of the culture conditions of the adipoblastic line 3T3L1, as a model of differentiated adipocytes in vitro. in 6 wells culture dishes, 300,000 cells were seed per well and grown with 10% fetal bovine serum supplemented culture media (by default, DMEM). The cells were maintained under controlled incubator conditions of 37°C, 5% CO2 and humidity. Once they reached confluence, they were deprived of serum for 16 hous. The product was added as suspension, at different concentrations keeping the same final volume for 24 hours in the same controlled maintaining conditions. Afterwards, the cells were processed and complementary tested for viability and toxicity by two tests described in pharmacological and toxicological studies:

a) Trypan Blue exclusion, which differentiates live/dead cells and

b) Metabolic activity by cellular dyeing with MTT (3-(4,5-dimethylthiazol-2-yl)-2,5-diphenyltetrazo liumbromid).

Results are shown in **Figure 5.** The data show that GMC-1 is non toxic on cultured adipocytes.

**Example 3: FUNCTIONAL ASSAY OF GMC-1 ON ADIPOCYTES AND FAT DEPOTS EX VIVO.**

The increase of the adiposity and the fat accumulation is associated to several pathological conditions such as diabetes mellitus type 2 and metabolic syndrome, because the adipocytes of the accumulated fat have being related to chronic inflammation, which means that is producing lower protective hormones (as leptin, adiponectin) and increasing the production of pro-inflammatory hormones (or citokines) as interleukin- 6 (IL-6). [Luo L., Liu M.; Adipose tissue in control of metabolism. J Endocrinol. 2016 Dec;231(3):R77-R99.]

In cosmetic terms, the increase of the adipose tissue in the hypodermis may be gradually reflected by deformation of the skin brought about by the thickening of the hypodermis in which the subcutatenous adipose tissue is found, which can result in undesired skin appearance and cellulitis.

Taking into consideration the differentiation state of the adipocyte cell, the expression of the pro-adipogenic transcription factor Peroxisome proliferator-activated receptor $\gamma$ (PPAR$\gamma$) is crucial. Another aspect is that increased expression of the insulin-dependent glucose transporter 4 (GLUT4) is related with improved insulin sensitivity.

Thus, an increase in PPAR$\gamma$ expression in WAT means increased pro-adiposity state and increased GLUT4 in WAT means decreased insulin resistance

We studied in vitro and ex vivo the capacity of GMC-1 to modify the phenotype of the adipose tissue.

The functional study is based in the phenotype plasticity of the adipocytes, as introduced in this section. Thus, we determined the expression of the genes such as interleukin-6 (IL-6), peroxisome proliferator-activated receptor-gamma (PPAR$\gamma$) and Glucose transporter 4 (GLUT4) [Jung, U.J. and Choi, M.S. Obesity and its metabolic complications: the role of adipokines and the relationship between obesity, inflammation, insulin resistance, dyslipidemia and nonalcoholic fatty liver disease. Int J Mol Sci. 2014 Apr 11;15(4):6184-223; ;Armoni, M. et al. Transcriptional regulation of the GLUT4 gene: from PPAR-gamma and FOXO1 to FFA and inflammation. Trends Endocrinol Metab. 2007 Apr;18(3):100-7.]

After the treatment (24 h duration) with the convenient suspension of non-toxic concentration 2x GMC-1(20 microg/ml) or plain vehicle as control over two models. First, the use of confluent fully differentiated adipocytes, obtained by manipulation of the culture conditions of an adipoblastic line, 3T311. Second the use of similar weight and mechanically disgregated sections of the visceral depot (epididimal region) from normo-weight rodents (*Rattus norvegicus*) male or female, 3-8 months old between 300-500 body weight (n=18) [Gao, X. et al. Decreased lipogenesis in white adipose tissue contributes to the resistance to high fat diet-induced obesity in phosphatidylethanolamine N-methyltransferase-deficient mice. Biochimica et Biophysica Acta 1851 (2015) 152-162]. The expression change was determined using Reverse transcription- quantitative polymerase chain reaction (RT-qPCR). 100 ng of converted DNA (cDNA) of the original mRNA extracted from the different samples were amplified with commercial gene expression assays (TaqMan, Life Technologies). The values were normalized against an endogenous control (beta-actin) and then the fold change in the expression was performed using the method 2^-deltadeltaCt.

Results are shown in **Figure 6**. GMC-1 modifies the phenotype plasticity of the adipocytes both *in vitro* and *ex vivo*.

## Claims

1. A graphene nanomaterial selected from graphene nanofibers, graphene oxide or reduced graphene oxide, or combinations thereof, wherein the graphene nanomaterial has a particle size distribution having a dn(90) of 0.60 $\mu$m or smaller in number of particle and a dv(90) of 80.00 $\mu$m or smaller in volume particle, as measured by laser diffraction particle analyzer.

2. The graphene nanomaterial according to claim 1, wherein the nanomaterial is in the form of graphene nanofibers.

3. The graphene nanomaterial according to claim 1 or 2, wherein its BET specific surface area is comprised between 100 and 500 $m^2$/g.

4. The graphene nanomaterial as defined in claim 3, wherein its BET specific surface area is between 300-350 $m^2$/g.

5. The graphene nanomaterial as defined in claim 1 or 2, wherein its pore volume is between 0.35-0.40 $cm^3$/g.

6. The graphene nanomaterial as defined in claim 1 or 2 wherein its BET specific surface area is between 300-350 $m^2$/g and its pore volume is between 0.35-0.40 $cm^3$/g.

7. The graphene nanomaterial according to any of claims 1-6, wherein the impurities in the nanomaterial are less than 0.01% by weight.

8. Use of a graphene nanomaterial according to any of claims 1 to 7 as a cosmetic.

9. Cosmetic use according to claim 8 to treat the skin.

10. Cosmetic use according to claim 8 or 9 to reduce adipose tissue of the skin, preferably to reduce fat in the hypodermis.

11. Cosmetic treatment method for preventing or reducing the increase in the volume of adipose tissue and/or the formation of fatty lumps, **characterized in that** the graphene nanomaterial according to any of claims 1-7, or a composition comprising it, is applied to the skin.

12. Cosmetic treatment according to claim 11 for the reduction of cellulitis, wrinkles and/or varicose veins.

13. A cosmetic composition comprising a graphene nanomaterial product as defined in any of claims 1 to 7 and a cosmetically acceptable excipient.

14. A cosmetic composition according to claim 13 suitable for topical use.

15. A cosmetic composition according to claims 13 or 14 wherein the composition is in a form selected from a cream, a lotion, an ointment, a microemulsion, a fatty ointment, a gel, an emulsion-gel, a paste, a foam, a tincture, a patch, a bandage, a membrane an and transdermal therapeutic systems.

16. A cosmetic composition according to claims 15 wherein the composition is in a form selected from a cream, a hydrogel or an emulsion-gel.

17. A method to prepare the product as defined in any of claims 1-7 from raw graphene nanofibers, graphene oxide or reduced graphene oxide, comprising the steps of:

a) purifying the raw graphene material, preferably using a strong acid, to remove any metal or impurity present in the graphene raw material,

b) Reducing the particle size of the purified graphene nanomaterial, preferably through an exfoliation process, to a particle size distribution having a dn(90) of 0.60 $\mu$m or smaller in number of particle and a dv(90) of 80.00 $\mu$m or smaller in volume particle, as measured by laser diffraction particle analyzer

c) Optionally subjecting the obtained product to a depyrogenization process.

**Figure 1**

**Figure 2**

**Figure 3A**

**Figure 3B**

## Figure 4A

d(0.1): 1.121   um          d(0.5): 1.573   um          d(0.9): 3.909   um

Particle Size Distribution

—GMC1, miércoles, 19 de julio de 2017 13:33:48     —GNFs, miércoles, 19 de julio de 2017 13:30:06

## Figure 4B

d(0.1): 19.764   um          d(0.5): 57.711   um          d(0.9): 103.114   um

Particle Size Distribution

—GMC1, miércoles, 23 de mayo de 2018 12:18:43     —GNFs, miércoles, 23 de mayo de 2018 11:51:44

Figure 5

Figure 6

Europäisches
Patentamt

European
Patent Office

Office européen
des brevets

# EUROPEAN SEARCH REPORT

Application Number

EP 18 38 2533

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | LINGLING OU ET AL: "Toxicity of graphene-family nanoparticles: a general review of the origins and mechanisms", PARTICLE AND FIBRE TOXICOLOGY, vol. 13, no. 1, 31 October 2016 (2016-10-31), pages 1-24, XP55534278, DOI: 10.1186/s12989-016-0168-y * abstract * * page 6, Table 1 * | 1-17 | INV. C01B32/18 C01B32/182 C01B32/198 A61K8/04 |
| A,D | SANGILIYANDI GURUNATHAN ET AL: "Synthesis, toxicity, biocompatibility, and biomedical applications of graphene and graphene-related materials", INTERNATIONAL JOURNAL OF NANOMEDICINE, 1 May 2016 (2016-05-01), pages 1927-1945, XP55534241, DOI: 10.2147/IJN.S105264 * pages 1938-1939, "Conclusion and future perspectives" * | 1-17 | |
| A | XIAOQING GUO ET AL: "Assessment of the toxic potential of graphene family nanomaterials", JOURNAL OF FOOD AND DRUG ANALYSIS, vol. 22, no. 1, 4 February 2014 (2014-02-04), pages 105-115, XP55534330, TW ISSN: 1021-9498, DOI: 10.1016/j.jfda.2014.01.009 * page 108 to page 112: "4.2. In vitro mammalian cell toxicity" & "4.3. In vivo toxicity." * | 1-17 | TECHNICAL FIELDS SEARCHED (IPC) C01B A61Q A61K |

-/--

The present search report has been drawn up for all claims

| Place of search | Date of completion of the search | Examiner |
|---|---|---|
| The Hague | 14 December 2018 | Rigondaud, Bernard |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

## EUROPEAN SEARCH REPORT

Application Number

EP 18 38 2533

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | MOHAMED H. LAHIANI ET AL: "Graphene and carbon nanotubes activate different cell surface receptors on macrophages before and after deactivation of endotoxins : Activation of macrophages before and after deactivation of endotoxin", JOURNAL OF APPLIED TOXICOLOGY., vol. 37, no. 11, 1 November 2017 (2017-11-01), pages 1305-1316, XP55534313, GB ISSN: 0260-437X, DOI: 10.1002/jat.3477 * the whole document * ----- | 8-17 | |
| A | DATABASE WPI Week 201772 Thomson Scientific, London, GB; AN 2017-66663N XP002787458, & KR 2017 0106062 A (BIOGENICS INC) 20 September 2017 (2017-09-20) * abstract * ----- | 1,8, 13-16 | |
| A | ANDREA FRANCESCO VERRE ET AL: "Improving the glial differentiation of human Schwann-like adipose-derived stem cells with graphene oxide substrates", INTERFACE FOCUS, vol. 8, no. 3, 6 June 2018 (2018-06-06), pages 1-7, XP55535044, GB ISSN: 2042-8898, DOI: 10.1098/rsfs.2018.0002 * abstract * * page 2, "2. Material and methods" * ----- -/-- | 1,8-11, 17 | TECHNICAL FIELDS SEARCHED (IPC) |

The present search report has been drawn up for all claims

| Place of search | Date of completion of the search | Examiner |
|---|---|---|
| The Hague | 14 December 2018 | Rigondaud, Bernard |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

**EUROPEAN SEARCH REPORT**

Application Number

EP 18 38 2533

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | MARTIN-GULLON I ET AL: "Differences between carbon nanofibers produced using Fe and Ni catalysts in a floating catalyst reactor", CARBON, ELSEVIER, OXFORD, GB, vol. 44, no. 8, 1 July 2006 (2006-07-01), pages 1572-1580, XP025010855, ISSN: 0008-6223, DOI: 10.1016/J.CARBON.2005.12.027 [retrieved on 2006-07-01] * page 1573; figure 1 * ----- | 1,15 | |
| | | | TECHNICAL FIELDS SEARCHED (IPC) |

The present search report has been drawn up for all claims

| Place of search | Date of completion of the search | Examiner |
|---|---|---|
| The Hague | 14 December 2018 | Rigondaud, Bernard |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 18 38 2533

This annex lists the patent family members relating to the patent documents cited in the above-mentioned European search report.
The members are as contained in the European Patent Office EDP file on
The European Patent Office is in no way liable for these particulars which are merely given for the purpose of information.

14-12-2018

| Patent document cited in search report | Publication date | Patent family member(s) | Publication date |
|---|---|---|---|
| KR 20170106062 A | 20-09-2017 | NONE | |

For more details about this annex : see Official Journal of the European Patent Office, No. 12/82

EPO FORM P0459

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20060134096 A
- EP 313353 A
- US 20140120081 A
- GB 2532449 A

**Non-patent literature cited in the description**

- **GURANATHAN S. ; KIM J-H.** *International Journal of Nanomedicine,* 2016, vol. 11, 1927-1945
- **SUTTER, P.** Epitaxial graphene: How silicon leaves the scene. *Nature Materials,* 2009, vol. 8 (3), 171-172
- **JACOBBERGER, R.M. et al.** Simple Graphene Synthesis via Chemical Vapor Deposition. *Journal of Chemical Education,* 2015, vol. 92 (11), 1903-1907
- **LAVIN-LOPEZ, M.P. et al.** Thickness control of graphene deposited over polycrystalline nickel. *New Journal of Chemistry,* 2015, vol. 39 (6), 4414-4423
- **GEIM, A.K. ; K.S. NOVOSELOV.** The rise of graphene. *Nature Materials,* 2007, vol. 6 (3), 183-191
- **LOTYA, M. et al.** Liquid phase production of graphene by exfoliation of graphite in surfactant/water solutions. *Journal of the American Chemical Society,* 2009, vol. 131 (10), 3611-3620
- **CHUA, C.K. ; M. PUMERA.** Chemical reduction of graphene oxide: A synthetic chemistry viewpoint. *Chemical Society Reviews,* 2014, vol. 43 (1), 291-312
- **CHUA, C.K. et al.** Graphite oxides: Effects of permanganate and chlorate oxidants on the oxygen composition. *Chemistry - A European Journal,* 2012, vol. 18 (42), 13453-13459
- **MARCANO, D.C. et al.** Improved synthesis of graphene oxide. *ACS Nano,* 2010, vol. 4 (8), 4806-4814
- **LAVIN-LOPEZ, M.D.P. et al.** Influence of different improved hummers method modifications on the characteristics of graphite oxide in order to make a more easily scalable method. *Industrial and Engineering Chemistry Research,* 2016, vol. 55 (50), 12836-12847
- **LAVIN-LOPEZ, M.P. et al.** Influence of the reduction strategy in the synthesis of reduced graphene oxide. *Advanced Powder Technology,* 2017, vol. 28 (12), 3195-3203
- **MCALLISTER, M.J. et al.** Single sheet functionalized graphene by oxidation and thermal expansion of graphite. *Chemistry of Materials,* 2007, vol. 19 (18), 4396-4404
- **ZHU, Y. et al.** Microwave assisted exfoliation and reduction of graphite oxide for ultracapacitors. *Carbon,* 2010, vol. 48 (7), 2118-2122
- **ZHANG, Y. et al.** Direct imprinting of microcircuits on graphene oxides film by femtosecond laser reduction. *Nano Today,* 2010, vol. 5 (1), 15-20
- **DEMAZEAU, G.** Solvothermal processes: A route to the stabilization of new material. *Journal of Materials Chemistry,* 1999, vol. 9 (1), 15-18
- **GAO, W. et al.** New insights into the structure and reduction of graphite oxide. *Nature Chemistry,* 2009, vol. 1 (5), 403-408
- **MARTIN-GULLON, I. et al.** Differences between carbon nanofibers produced using Fe and Ni catalysts in a floating catalyst reactor. *Carbon,* 2006, vol. 44 (8), 1572-1580
- **ZHANG, L. et al.** A review: Carbon nanofibers from electrospun polyacrylonitrile and their applications. *Journal of Materials Science,* 2014, vol. 49 (2), 463-480
- Harry's Cosmeticology. Chemical Publishing Co, 2015
- **SAPONARO C et al.** *Nutrients,* 2015, vol. 7, 9453-9474
- **TONTONOZ P. et al.** *Annu. Rev. Biochem.,* 2008, vol. 77, 289-312
- **LUO L. ; LIU M.** Adipose tissue in control of metabolism. *J Endocrinol.,* December 2016, vol. 231 (3), R77-R99
- **JUNG, U.J. ; CHOI, M.S.** Obesity and its metabolic complications: the role of adipokines and the relationship between obesity, inflammation, insulin resistance, dyslipidemia and nonalcoholic fatty liver disease. *Int J Mol Sci.,* 11 April 2014, vol. 15 (4), 6184-223
- **ARMONI, M. et al.** Transcriptional regulation of the GLUT4 gene: from PPAR-gamma and FOXO1 to FFA and inflammation. *Trends Endocrinol Metab.,* April 2007, vol. 18 (3), 100-7

• **GAO, X. et al.** Decreased lipogenesis in white adipose tissue contributes to the resistance to high fat diet-induced obesity in phosphatidylethanolamine N-methyltransferase-deficient mice. *Biochimica et Biophysica Acta,* 2015, vol. 1851, 152-162